(19) Europäisches Patentamt
European Patent Office
Office européen des brevets

(11) Veröffentlichungsnummer: **0 223 037 A1**

(12) # EUROPÄISCHE PATENTANMELDUNG

(21) Anmeldenummer: **86113774.3**

(22) Anmeldetag: **04.10.86**

(51) Int. Cl.⁴: **C07D 239/62**, //C07C95/08,C07C45/56

(30) Priorität: **24.10.85 CH 4592/85**

(43) Veröffentlichungstag der Anmeldung: **27.05.87 Patentblatt 87/22**

(84) Benannte Vertragsstaaten: **AT BE CH DE FR GB IT LI NL**

(71) Anmelder: **F. HOFFMANN-LA ROCHE & CO. Aktiengesellschaft**

**CH-4002 Basel(CH)**

(72) Erfinder: **Kägi, Dieter, Dr.**
**Grenzacherstrasse 477**
**CH-4058 Basel(CH)**

(74) Vertreter: **Grossner, Lutz, Dr. et al**
**Grenzacher Strasse 124 Postfach 3255**
**CH-4002 Basel(CH)**

(54) **Phenylbarbitursäuren und deren Herstellung.**

(57) Die neuen Verbindungen der Formel

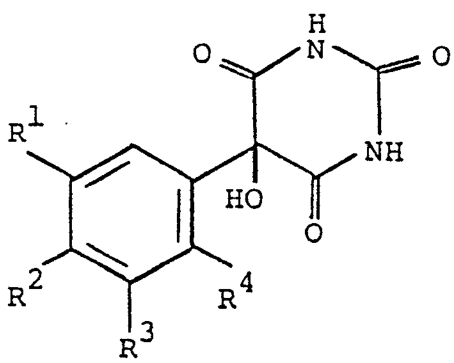

I

worin $R^1$ und $R^3$ nieder-Alkoxy oder nieder-Alkylthio; $R^2$ nieder-Alkoxy, Hydroxy oder Amino; und $R^4$ Wasserstoff; oder $R^1$ und $R^2$ nieder-Alkoxy, $R^3$ Wasserstoff und $R^4$ nieder-Alkyl bedeuten,
sind Zwischenprodukte bei der Herstellung von Benzylpyrimidinen. Man erhält die Verbindungen I durch Umsetzung eines durch $R^1$-$R^4$ entsprechend substituierten Benzols mit Alloxan.

EP 0 223 037 A1

Xerox Copy Centre

## Phenylbarbitursäuren und deren Herstellung

Die vorliegende Erfindung betrifft neue substituierte Phenylbarbitursäuren der Formel

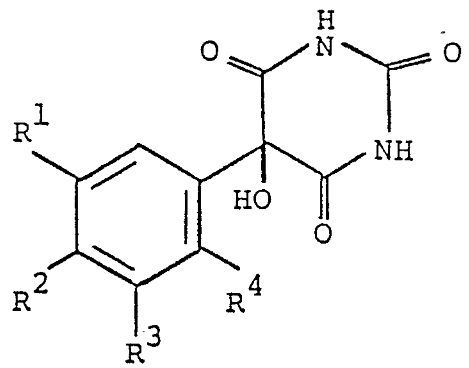

I

worin $R^1$ und $R^3$ nieder-Alkoxy oder nieder-Alkylthio; $R^2$ nieder-Alkoxy, Hydroxy oder Amino; und $R^4$ Wasserstoff; oder $R^1$ und $R^2$ nieder-Alkoxy, $R^3$ Wasserstoff und $R^4$ nieder-Alkyl bedeuten.

Niedere Alkoxy-und Alkylreste sind vorzugsweise solche mit bis zu 7 C-Atomen. Sie können geradkettig oder verzweigt sein. Beispiele solcher Reste sind Methoxy, Aethoxy und Propoxy bzw. Methyl, Aethyl und Propyl. Bevorzugte Verbindungen der Formel I sind diejenigen, in denen $R^1$, $R^2$ und $R^3$ Methoxy und $R^4$ Wasserstoff; oder $R^1$ und $R^3$ Methoxy, $R^2$ Amino und $R^4$ Wasserstoff; oder $R^1$ und $R^2$ Methoxy, $R^3$ Wasserstoff und $R^4$ Methyl sind.

Die Verbindungen der Formel I werden erfindungsgemäss dadurch hergestellt, dass man eine Verbindung der Formel

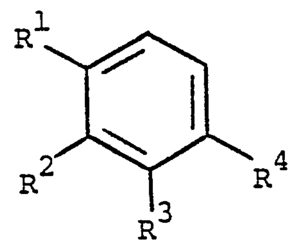

II

in der $R^1$ bis $R^4$ die obige Bedeutung haben, mit Alloxan umsetzt.

Die Umsetzung wird zweckmässig so vorgenommen, dass man zu einer wässrigen Alloxanlösung eine Verbindung der Formel II gibt und das Reaktionsgemisch intensiv rührt. Gegebenenfalls kann dem Reaktionsgemisch ein Friedel-Crafts-Katalysator, z.B. Zinkchlorid, zugesetzt werden. Die Verbindung der Formel I scheidet sich in fester Form ab und kann abfiltriert und gegebenenfalls durch Umkristallisation gereinigt werden.

Die Verbindungen der Formel I können durch Hydrolyse, z.B. durch Behandlung mit wässriger Schwefelsäure unter Erwärmen, Neutralisation des Hydrolysats und Extraktion mit einem geeigneten organischen Lösungsmittel in Aldehyde der Formel

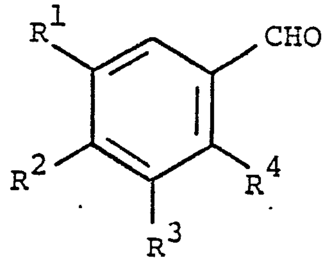

III

in der $R^1$-$R^4$ die obige Bedeutung haben, übergeführt werden.

Die Aldehyde der Formel III sind bekannte Verbindungen, die als Zwischenprodukte bei der Herstellung von antibakteriell wirksamen Benzylpyrimidinen, wie Trimethoprim, Verwendung finden können. Die vorliegende Erfindung eröffnet einen technisch vorteilhaften Zugang zu diesen Verbindungen.

Die Erfindung wird durch das nachstehende Beispiel weiter erläutert.

Beispiel

Zu einer Lösung von 35 g Alloxanthetrahydrat in 120 ml Wasser wurden 25 g 2,6-Dimethoxyanilin gegeben. Unter starkem Rühren ging das anfänglich heterogene Gemisch nach 1 Stunde in eine braune Lösung über, aus welcher sind nach kurzer Zeit graue Kristalle abschieden. Das Reaktionsgemisch wurde 24 Stunden bei Raumtemperatur weitergerührt, bis gemäss Dünnschichtchromatogramm (Cyclohexan/Essigester/Wasser = 60:30:1) kein Dimethoxyanilin mehr nachweisbar war. Das Reaktionsgemisch wurde abgenutscht, der Nutschrückstand wurde mit 4 Portionen zu 80 ml, Wasser aufgeschlämmt und trocken gesaugt. Der Feststoff wurde 16 Stunden bei 100°/16 mbar getrocknet. Man erhielt 44,2 g 5-(4-Amino-3,5-dimethoxyphenyl)-5-hydroxybarbitursäure als hellgraues Pulver vom Smp. 240-243° - (unter Zers.). Zur Reinigung kann das Präparat aus 1-Butanol umkristallisiert werden, wobei ein Produkt vom Smp. 242-244° (Zers.) erhalten wird.

Die 5-(4-Amino-3,5-dimethoxyphenyl)-5-hydroxybarbitursäure kann wie folgt in den entsprechenden Aldehyd der Formel III übergeführt werden:

28 ml Schwefelsäure (d = 1,8; 0,45 Mol) werden unter Rühren auf 120° erwärmt und in einer Portion mit 11,25 g 5-(4-Amino-3,5-dimethoxyphenyl)-5-hydroxybarbitursäure (0,038 Mol) versetzt. Das Gemisch wird gerührt, bis keine Gasentwicklung mehr zu beobachten ist (ca. 15-20 Minuten) und dann unter Rühren in 140 g Eis und 140 g Wasser gegossen. Ev. entstandene harzige Polymerisationsprodukte werden durch eine Filtration über ein Polster von 15 g Celite abgetrennt. Unter Rühren und Kühlen wird das Filtrat mit 63,6 g Kaliumcarbonatpulver versetzt, bis die Lösung neutral ist. Die wässrige Suspension wird mit dreimal 50 ml Aethylacetat extrahiert, die vereinigten organischen Phasen über Natriumsulfat getrocknet und im Wasserstrahlvakuum eingeengt. Der Rückstand wird im Hockvakuum bis zur Gewichtskonstanz getrocknet. Man erhält 6,36 g rohen 4-(N,N-Dimethylamino)-3,5-dimethoxybenzaldehyd als gelbes Oel. Die gaschromatographisch ermittelte Reinheit beträgt 96%.

**Ansprüche**

1. Verbindungen der Formel

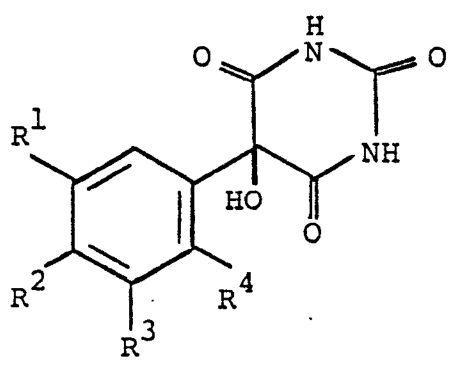

I

worin $R^1$ und $R^3$ nieder-Alkoxy oder nieder-Alkylthio; $R^2$ nieder-Alkoxy, Hydroxy oder Amino; und $R^4$ Wasserstoff; oder $R^1$ und $R^2$ nieder-Alkoxy, $R^3$ Wasserstoff und $R^4$ nieder-Alkyl bedeuten.

2. 5-(4-Amino-3,5-dimethoxyphenyl)-5-hydroxybarbitursäure.

3. Verfahren zur Herstellung von Verbindungen der Formel I von Anspruch 1, dadurch gekennzeichnet, dass man eine Verbindung der Formel

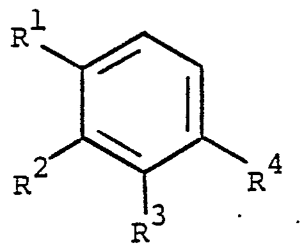

II

in der $R^1$-$R^4$ die in Anspruch 1 angegebene Bedeutung haben, mit Alloxan umsetzt.

Europäisches Patentamt

# EUROPÄISCHER RECHERCHENBERICHT

Nummer der Anmeldung

EP 86 11 3774

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-C- 107 720 (C.F. BOEHRINGER & SÖHNE)<br>* gesamtes Dokument *<br>--- | 1,3 | C 07 D 239/62 //<br>C 07 C 95/08<br>C 07 C 45/56 |
| A | DE-C- 113 722 (C.F. BOEHRINGER & SÖHNE)<br>* gesamtes Dokument *<br>--- | 1,3 | |
| A | BERICHTE DER DEUTSCHEN CHEMISCHEN GESELLSCHAFT, Band 56, 1923, Seiten 2464-2468, Verlag Chemie GmbH, Leipzig; T. SZEKI "Über einige Kondensationsprodukte des Oxyhydrochinon-trimethyläthers"<br>* Seite 2466, Verbindung III *<br>--- | 1,3 | |
| | | | **RECHERCHIERTE SACHGEBIETE (Int. Cl.4)** |
| A | DE-C- 112 174 (C.F. BOEHRINGER & SÖHNE)<br>* Seite 3, Verbindungen 10,12 *<br>--- | 1-3 | C 07 D 239/00<br>C 07 C 45/00<br>C 07 C 47/00<br>C 07 C 95/00 |
| A | CHEMICAL ABSTRACTS, Band 100, Nr. 25, 18. Juni 1984, Seite 590, Spalte 2, Abstract Nr. 209746A; Columbus, Ohio, US; W.E. ADAMS et al.: "Alloxan analogs as potential pancreatic-imaging radiopharmaceuticals", & J. PHARM. SCI. 1984, 73(3), 394-396<br>--- -/- | 1-3 | |

Der vorliegende Recherchenbericht wurde für alle Patentansprüche erstellt.

| Recherchenort | Abschlußdatum der Recherche | Prüfer |
|---|---|---|
| BERLIN | 26-01-1987 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

EP 86 11 3774

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | HOUBEN-WEYL, Methoden der organischen Chemie, Band VII, Teil 1, Sauerstoff-Verbindungen II, 1954, Georg Thieme Verlag, Stuttgart; O. BAYER "Aldehyde" * Seiten 312,313 * | 1-3 | |
| | --- | | |
| A | DE-C- 108 026 (C.F. BOEHRINGER & SÖHNE) | | |
| | ----- | | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| BERLIN | 26-01-1987 | VAN AMSTERDAM L.J.P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82